# EUROPÄISCHE PATENTSCHRIFT

(11) **EP 2 470 117 B1**
(45) Veröffentlichungstag und Bekanntmachung des Hinweises auf die Patenterteilung: **27.04.2016**
(21) Anmeldenummer: 10749470.0
(22) Anmeldetag: 25.08.2010
(51) Int. Cl.: A61F 2/16, A61F 2/00, A61L 31/08

(54) **INTRAOKULARLINSE**
INTRAOCULAR LENS
LENTILLE INTRAOCULAIRE

(30) Priorität: 28.08.2009 DE 202009011716 U
(43) Veröffentlichungstag der Anmeldung: 04.07.2012
(73) Patentinhaber: MIRO GmbH, 81241 München (DE)
(72) Erfinder: MÜLLER-LIERHEIM, Wolfgang, G., K., 81243 München (DE)
(74) Vertreter: Reichert & Lindner Partnerschaft Patentanwälte
(86) Internationale Anmeldenummer: PCT/EP2010/005212
(87) Internationale Veröffentlichungsnummer: WO 2011/023380

(56) Entgegenhaltungen:
- EP-A1- 2 067 453
- DE-A1- 10 351 469
- US-A- 5 094 876
- US-A1- 2002 133 167

## Beschreibung

Die Erfindung betrifft eine Intraokularlinse. Derartige Linsen werden insbesondere nach Eintrübung der natürlichen Augenlinse als Ersatz der natürlichen Augenlinse im Rahmen einer Kataraktoperation implantiert. In herkömmlicher Weise besteht der Linsenkörper aus hydrophobem Material, insbesondere Copolymeren, welche Acrylat und/oder Methacrylat enthalten. Zur Herabsetzung der Klebrigkeit ist es auch bekannt, dem Linsenmaterial fluoriertes Acrylat oder Methacrylat zuzusetzen (WO 2007/062864). Ferner ist es bekannt für die Intraokularlinse ein Linsenmaterial zu verwenden, welches einen hohen Brechungsindex aufweist um eine geringe Linsendicke zu ermöglichen. Derartige insbesondere falt- oder rollbare Linsen können dann durch relativ kleine Schnitte mit Hilfe von Injektoren, wie sie beispielswiese aus US 6 355 046 B2 bekannt sind, in das Auge implantiert werden. Aus US 2002/ 0133 167 A1 ist ein Injektor mit hydrophiler Innenbeschichtung bekannt.

Aus DE 103 51469 A1 ist eine Intraokularlinse für die Katarakt-Chirurgie bekannt welche aus einem hydrophoben Material, insbesondere Silikon, besteht und deren Obefläche zur Reduzierung der Nachstarrrate eine hydrophile Hyaluronanschicht aufweist.

Aus EP 2 067 453 A1 ist eine Keratoprothese bekannt, welche bei schwerwiegenden Hornhautverletzungen in die Hornhaut des Patienten implantiert wird. Die Keratoprothese besitzt einen zylindrischen optischen Teil, dessen dem Augenlid zugewandte Oberfläche zur guten Benetzbarkeit im Kontakt zum Tränenfilm hydrophil ausgebildet ist.

Aufgrund des Brechungsindex-Unterschiedes des Linsenmaterials der implantierten Intraokularlinse gegenüber dem im Auge umgebenden Medium, insbesondere dem Kammerwasser in der Vorderkammer des Auges und dem Glaskörper an der Linsenrückseite, kommt es an den Grenzflächen zu Lichtreflexion. Diese ist umso ausgeprägter je höher der Brechungsindex-Unterschied zwischen dem Linsenmaterial und dem umgebenden Medium ist.

Außerdem werden bei der Herstellung der Intraokularlinsen die Oberflächen mittels Submikrometer-Technologie spanabhebend bearbeitet um einen anschließenden Polierschritt zu vermeiden. Die so behandelten Linsenoberflächen weisen jedoch noch Mikrorauigkeiten auf, die zu Lichtstreuung beitragen können.

Aufgabe der Erfindung ist es eine Intraokularlinse zu schaffen, bei welcher ein möglichst kontinuierlicher lichtoptischer Übergang zwischen dem die im Auge implantierte Linse umgebenden Medium und der Linsenoberfläche stattfindet.

Diese Aufgabe wird durch die Intraokularlinse mit den Merkmalen des Schutzanspruches 1 gelöst.

Eine derartige Linse besitzt einen hydrophoben Linsenkörper an dessen Oberfläche eine hydrophile Schicht vorgesehen ist. Bevorzugt besteht der Linsenkörper aus einem hydrophoben und faltbaren Polymermaterial aus mindestens einem Acrylat und/oder Methacrylat, zur Herabsetzung der Klebrigkeit kann ein fluoriertes Acrylat oder Methacrylat zugesetzt sein. Geeignete Linsenmaterialien sind beispielsweise aus WO 2007/062864 A2 bekannt ist. Ferner kann als hydrophobes Material für den Linsenkörper Silikonkautschuk verwendet werden.

Die hydrophile Schicht besteht aus wenigstens einem hydrophilen Acrylat mit guter Gewebe- und Blutverträglichkeit. Diese Beschichtung verhindert das Anheften von Fibrin und adhärenten Zellen und wirken dadurch einer postoperativen Membranbildung (sekundär Katarakt, PCO = Posterior Capsular Opacification) entgegen.

Bei dem hydrophoben Linsenmaterial handelt es sich um ein solches, welches weniger als 5 Vol% Wasser aufnimmt. Es handelt sich um falt- oder rollbare Linsenkörper.

Bei den Linsenkörpern kann es sich um solche handeln, deren Oberfläche mittels Sub-mikrometer-Technologie spanabhebend hergestellt sind und somit Mikrorauigkeiten aufweisen, die zu Lichtstreuung beitragen können. Anstelle der spanabhebenden Bearbeitung können die Linsenkörper, insbesondere die aus Silikonkautschuk bestehenden Linsenkörper im Molding-Verfahren hergestellt werden. Dabei überträgt sich die Rauigkeit der Mold-Oberflächen auf die Linsenoberfläche. Durch Aufbringen der hydrophilen Beschichtung auf diese Oberfläche werden die Rauigkeiten geglättet und Lichtstreuung weitestgehend vermieden.

Der Brechungsindex der hydrophilen Beschichtung ist so gewählt, dass er zwischen dem Brechungsindex des Linsenmaterials und dem Brechungsindex des umgebenden Mediums im Auge, welches im Wesentlichen das Kammerwasser und der Glaskörper sind, liegt. Das bedeutet, dass der Brechungsindex vorzugsweise zwischen n=1,336 (Kammerwasser) bzw. 1,38 (Glaskörper) und n=1,56 (aus WO 2007/062864 A2 bekanntes Polymermaterial) vorzugsweise gewählt werden kann. Da die hydrophile Beschichtung, insbesondere hydrophile Acrylatschicht zwischen dem hydrophoben Linsenmaterial, aus dem der Linsenkörper besteht, und dem umgebenden Medium im Auge, nämlich in dem des Kammerwassers und des Glaskörpers liegt, erreicht man einen angenähert kontinuierlichen lichtoptischen Übergang zwischen dem Kammerwasser, dem Linsenkörper und dem Glaskörper, wodurch Lichtreflexion und durch die verbleibenden Mikrorauigkeiten verursachte Lichtstreuung reduziert werden oder sich vermeiden lassen.

Durch die hydrophile Beschichtung wird außerdem die Gleiteigenschaft der Linse beim Implantieren mit Hilfe eines Injektors oder anderem Implantierwerkzeug, z.B. Pinzette verbessert. Derartige Injektoren sind beispielsweise aus US 6 355 046 B2 bekannt und dienen zum Halten oder Rollen der zu implantierenden Linse. Beim Implantieren wird die gefaltete oder gerollte Linse durch eine Kanüle, welche durch eine minimale Öffnung im Auge gesteckt, ist in das Auge implantiert.

Da die Innenwandung der Kanüle und der Faltkartusche, welche bei den bekannten Injektoren vorgesehen sind, in der Regel aus Polyethylen oder Polypropylen bestehen ist die Gleiteigenschaft an der Innenwandung der Kartusche und der Kanüle relativ gering, sodass ohne zusätzliche Gleitmittel der Injiziervorgang erschwert ist. Die zur Anwendung kommenden Gleitmittel können dabei in das Auge gelangen.

Gemäß der Erfindung lassen sich diese Nachteile durch eine an der Innenwandung der Faltkartusche und der Kanüle aufgebrachten hydrophilen Acrylatschicht beseitigen.

Demgemäß schlägt die Erfindung auch ein Implantationsset vor, welches aus der oben beschriebenen Intraokularlinse und einem Injektor zum implantieren der Intraokularlinse besteht. Gegebenenfalls kann die Intraokularlinse im Injektor aufbewahrt werden, wobei der Injektor ein Einmalteil darstellt. Aufgrund der hydrophilen Beschichtung an der Intraokularlinse und/oder an der Innenwand der Injektionskanüle und ggf. an der Faltkartusche des Injektors erreicht man eine Verbesserung der Gleiteigenschaften und somit eine Erleichterung beim Implantieren der Intraokularlinse.

Anhand der Figuren wird an Ausführungsbeispielen die Erfindung noch erläutert. Es zeigt:
Figur 1 schematisch eine schnittbildliche Darstellung einer Intraokularlinse als Ausführungsbeispiel der Erfindung; und
Figur 2 ein Ausführungsbeispiel für einen Injektor, mit welchem eine Intraokularlinse in ein Auge implantiert werden kann.

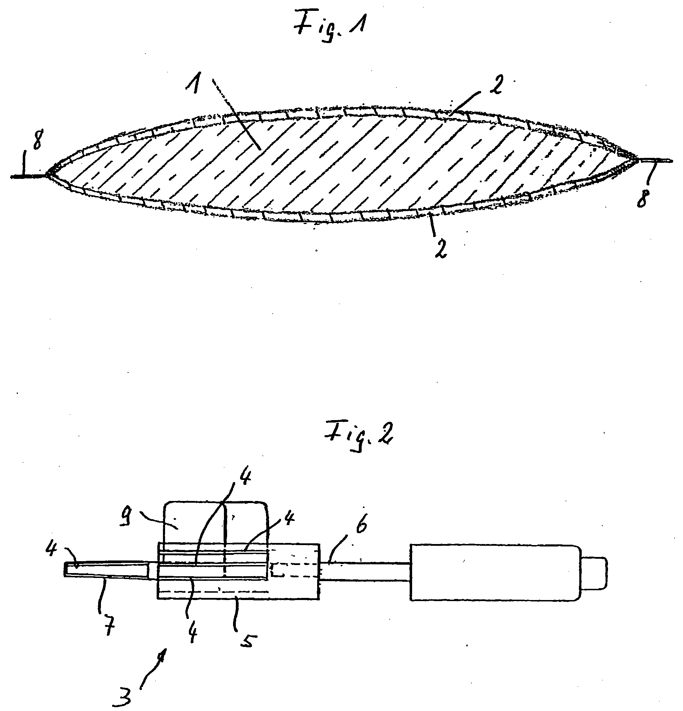

Das in der Figur 1 dargestellte Ausführungsbeispiel einer Intraokularlinse besitzt einen Linsenkörper 1 aus hydrophobem Material. Das hydrophobe Material kann aus einem Copolymermaterial gebildet sein, welches wenigstens ein Acrylat und/oder Methacrylat aufweist. Das Linsenmaterial ist so ausgebildet, dass der Linsenkörper 1 gefaltet oder gerollt werden kann. Die Oberfläche des Linsenkörpers 1 besitzt eine Mikrorauigkeit, welche aus dem spanabhebenden Herstellungsverfahren insbesondere nach Submikrometer-Technologie herrühren kann. Auf die mikroraue Oberfläche des Linsenkörpers 1 ist eine hydrophile Schicht 2 aus einem hydrophilen Acrylat aufgebracht. Bei diesem hydrophilen Acrylat kann es sich beispielsweise um Dihydroxypropylmethacrylat (DHPMA) handeln. Die Beschichtung erfolgt mit Hilfe eines noch nachstehend erläuterten Beschichtungsverfahrens. Am Rand des optischen Linsenkörpers kann eine Haptik 8 in Form von Fäden oder Stützstegen oder in Form einer den Linsenkörper 1 ganz oder teilweise umfassenden Stützumrandung vorgesehen sein.

Für diese Implantation einer derartigen Intraokularlinse eignet sich ein in herkömmlicher Weise ausgebildeter Intraokularlinsen-Injektor, wie er beispielsweise aus US 6 355 046 B2 bekannt ist.

Das in der Figur 2 dargestellte Ausführungsbeispiel eines Injektors besitzt im Wesentlichen einen herkömmlichen Aufbau. Dieser besteht aus einer Faltkartusche 5, in welcher die Intraokularlinse zunächst in ungefaltetem Zustand aufbewahrt werden kann. Für die Implantation wird die Linse in der Linsenkartusche 5 beispielsweise mit Hilfe zusammenklappbarer Flügel 9 gefaltet oder gerollt, sodass sich ihr Durchmesser auf den Innendurchmesser einer Kanüle 7 verringert. Mit Hilfe eines Stößels 6 wird die gefaltete oder gerollte Linse aus der Kartusche 5 durch die Kanüle 7, welche durch eine kleine inzision am Auge geführt ist, hindurchgeschoben und in das Auge implantiert. Dabei wird die Intraokularlinse in die vorher eröffnete Linsenkapsel eingesetzt. Zur Verbesserung der Gleiteigenschaften sind die Innenwandungen der Faltkartusche 5, welche mit der Oberfläche der Linse in Berührung kommen, sowie die Innenwand der Kanüle 7 mit einer hydrophilen Beschichtung 4, welche identisch mit der hydrophilen Beschichtung 2 der Intraokularlinse ist, ausgestattet.

Im Folgenden wird ein Ausführungsbeispiel der erfindungsgemäßen Intraokularlinse noch näher erläutert.

Auf den Linsenkörper 1 aus einem hydrophoben Material, beispielsweise einem aus WO 2007/062864 A2 bekannten hydrophoben Linsenmaterial, wird nach Aktivierung der Oberfläche des Linsenkörpers 1 die hydrophile Schicht 2 aufgebracht. Die Aktivierung der Oberfläche des Linsenkörpers 1 kann durch Verseifen, beispielsweise mit Natriumhydroxid erfolgen, wodurch die Oberfläche des Linsenkörpers negativ aufgeladen wird. Die Aktivierung der Oberfläche kann auch durch Plasmaaktivierung, beispielsweise unter Verwendung eines Stickstoff-Plasmas erfolgen. Auf die aktivierte Oberfläche wird das Monomer des hydrophilen Acrylats beispielsweise Dihydroxypropylmethacrylat (DHPMA) mit einem Lichtinitiator, insbesondere UV-Initiator beispielsweise mittels Ethylacetat in Lösung aufgebracht. Als weiteres Lösungsmittel eignet sich Tetrahydrofuran. Nach Abdampfen des Lösungsmittels erfolgt die Polymerisation mit Hilfe von Bestrahlung, insbesondere UV-Bestrahlung. Anschließend wird die Oberfläche gewaschen und bei etwa 35°C vakuumgetrocknet.

Hierdurch entsteht in einem Oberflächenbereich des hydrophoben Linsenmaterials ein interpenetrierendes Polymer-Netzwerk. Es entsteht insbesondere bei oberflächlichem Anquellen des hydrophoben Linsenmaterials durch das Lösungsmittel, mit welchem das hydrophile Acrylat als Schicht auf die Oberfläche des Linsenkörpers aufgebracht wird. Dabei werden Moleküle des hydrophile Acrylats in die Oberfläche des hydrophoben Linsenmaterials eingelagert und nach Polymerisation mit diesem vernetzt. Durch die oben beschriebene Aktivierung der Oberfläche des Linsenkörpers wird dieser Vorgang noch unterstützt. Hierdurch entsteht an der Oberfläche des Linsenkörpers mit dem interpenetrierenden Netzwerk zwischen dem Kern des Linsenkörpers und der hydrophilen Schicht auf der Oberfläche des Linsenkörpers eine Zwischenschicht mit einem Brechungsindex der zwischen dem Brechungsindex der hydrophilen Schicht und dem Brechungsindex des Linsenkörpers liegt. Hierdurch wird im Gegensatz zu der aus US 2009/0018651 A1 bekannten Beschichtung aus einer oder mehreren Schichten zwischen dem Linsenkörpermaterial und der Beschichtung ohne zusätzliche Beschichtung ein reflektionsfreier Übergang zwischen Beschichtung und Linsenkörpermaterial erreicht.

Unterstützt wird die Bildung dieses interpenetrierenden Polymer-Netzwerks durch die beschriebene Aktivierung der Oberfläche und auch durch die Bestrahlung insbesondere UV-Bestrahlung. Es entsteht hierdurch ein permanentes Polymer-Netzwerk.

Eine weitere Verbesserung des kontinuierlichen Übergangs der Brechungsindizes zwischen dem Augenkammerwasser und dem hydrophoben Linsenkörper der Intraokularlinse lässt sich noch dadurch erreichen, dass das Material der hydrophilen Schicht vom Kammerwasser angequollen wird. Im angequollenen Oberflächenbereich der hydrophilen Schicht liegt ein Brechungsindex vor, der zwischen dem Brechungsindex des Kammerwassers und dem Brechungsindex der hydrophilen Schicht liegt. Somit wird ein reflektionsfreier Übergang zwischen dem Kammerwasser und der hydrophilen Schicht auf dem Linsenkörper erreicht.

In gleicher Weise können auch die Oberflächen des in der Figur 2 dargestellten Injektors mit der hydrophilen Beschichtung versehen werden.

### Bezugszeichenliste

- 1.: Hydrophober Linsenkörper
- 2.: Hydrophile Schicht
- 3.: IOL-Injektor
- 4.: Hydrophile Beschichtung
- 5.: Faltkartusche
- 6.: Stößel
- 7.: Kanüle
- 8.: Linsenhaptik
- 9.: Faltflügel

## Patentansprüche

1. Intraokularlinse, welche als Ersatz der natürlichen Augenlinse in ein Auge zu implantieren ist, mit einem falt- oder rollbaren hydrophoben Linsenkörper (1), und einer an der Oberfläche des Linsenkörpers (1) vorgesehenen hydrophilen Schicht (2), **dadurch gekennzeichnet, dass** der Brechungsindex der hydrophilen Schicht (2), zwischen dem Brechungsindex des hydrophoben Linsenkörpers (1) und dem Brechungsindex des Kammerwassers der Augenvorderkammer und/oder des Augenglaskörpers liegt.

2. Intraokularlinse nach Anspruch 1, **dadurch gekennzeichnet, dass** der Linsenkörper aus einem Polymermaterial besteht, das aus zumindest einem hydrophoben Acrylat und/oder hydrophoben Methacrylat oder aus Silikonkautschuk gebildet ist.

3. Intraokularlinse nach Anspruch 1 oder 2, **dadurch gekennzeichnet, dass** die hydrophile Schicht (2) aus hydrophilem Acrylat gebildet ist.

4. Intraokularlinse nach einem der Ansprüche 1 bis 3, **dadurch gekennzeichnet, dass** der Linsenkörper (1) an seiner Oberfläche eine aus der spanabhebenden Linsenherstellung oder Formgießen (Molding) der Linse resultierende Mikrorauigkeit aufweist.

5. Intraokularlinse nach einem der Ansprüche 1 bis 4, **dadurch gekennzeichnet, dass** in einer Oberflächenschicht des Linsenkörpers (1) Moleküle der hydrophilen Schicht eingelagert sind.

6. Intraokularlinse nach Anspruch 5, **dadurch gekennzeichnet, dass** die eingelagerten Moleküle der hydrophilen Schicht mit den Molekülen des Linsenkörperpolymers vernetzt sind.

7. Intraokularlinse nach Anspruch 5 oder 6, **dadurch gekennzeichnet, dass** die Oberflächenschicht des Linsenkörpers, in welchem die Moleküle der hydrophilen Schicht eingelagert sind, einen Brechungsindex zwischen den Brechungsindizes der hydrophilen Schicht (2) und des Linsenkörpers (1) aufweist.

8. Intraokularlinse nach einem der Ansprüche 1 bis 7, **dadurch gekennzeichnet, dass** die hydrophile Schicht (2) durch Augenkammerwasser anquellbar ist und der Brechungsindex des angequollenen Oberflächenbereichs der hydrophilen Schicht (2) zwischen den Brechungsindizes des Kammerwassers und der hydrophilen Schicht (2) liegt.

9. Intraokularlinse nach einem der Ansprüche 1 bis 8, **dadurch gekennzeichnet, dass** das hydrophile Acrylat Dihydropropylmethacrylat ist.

10. Injektorset, welches einen Injektor (3) aufweist, mit welchem eine Intraokularlinse nach einem der Ansprüche 1 bis 9 zu implantieren ist, wobei der Injektor zumindest an seiner Innenwand, entlang welcher die Intraokularlinse bei der Implantation geführt wird, eine hydrophile Beschichtung (4) aufweist, und die Intraokularlinse Bestandteil des Injektorsets ist.

11. Injektorset nach Anspruch 10, **dadurch gekennzeichnet, dass** die hydrophile Beschichtung eine Acrylatbeschichtung ist.

## Claims

1. An intraocular lens, which is to be implanted into an eye as a replacement of the natural eye lens, comprising a foldable or rollable hydrophobic lens body (1) and a hydrophilic layer (2) provided on the surface of the lens body (1), **characterized in that** the refractive index of the hydrophilic layer (2) is between the refractive index of the hydrophobic lens body (1) and the refractive index of the aqueous humor of the anterior chamber of the eye and/or of the vitreous body of the eye.

2. The intraocular lens according to claim 1, **characterized in that** the lens body is comprised of a polymer material which is formed from at least one hydrophobic acrylate and/or hydrophobic methacrylate or from silicone rubber.

3. The intraocular lens according to claim 1 or 2, **characterized in that** the hydrophilic layer (2) is formed from hydrophilic acrylate.

4. The intraocular lens according to any one of the claims 1 to 3, **characterized in that** the lens body (1) has at its surface a micro-roughness resulting from the cutting lens production or the molding of the lens.

5. The intraocular lens according to any one of the claims 1 to 4, **characterized in that** molecules of the hydrophilic layer are deposited in a surface layer of the lens body (1).

6. The intraocular lens according to claim 5, **characterized in that** the deposited molecules of the hydrophilic layer are cross-linked with the molecules of the lens body polymer.

7. The intraocular lens according to claim 5 or 6, **characterized in that** the surface layer of the lens body in which the molecules of the hydrophilic layer are deposited has a refractive index between the refractive indices of the hydrophilic layer (2) and the lens body (1).

8. The intraocular lens according to any one of the claims 1 to 7, **characterized in that** the hydrophilic layer (2) can be made to swell by means of the aqueous humor of the eye and the refractive index of a swollen surface region of the hydrophilic layer (2) is between the refractive indices of the aqueous humor and the hydrophilic layer (2).

9. The intraocular lens according to any one of the claims 1 to 8, **characterized in that** the hydrophilic acrylate is dihydropropyl methacrylate.

10. An injector set having an injector (3) with which an intraocular lens according to any one of the claims 1 to 9 is to be implanted, wherein the injector, at least on its inner wall along which the intraocular lens is guided during implantation, has a hydrophilic coating (4), and the intraocular lens is a component part of the injector set.

11. The injector set according to claim 10, **characterized in that** the hydrophilic coating is an acrylate coating.

## Revendications

1. Lentille intraoculaire, qui est à implanter dans un oeil en remplacement du cristallin naturel, comportant un corps de cristallin (1) hydrophobe pouvant être plié ou roulé, et une couche hydrophile (2) prévue sur la surface du corps de cristallin (1), **caractérisée en ce que** l'indice de réfraction de la couche hydrophile (2) est situé entre l'indice de réfraction du corps de cristallin (1) hydrophobe et l'indice de réfraction de l'humeur aqueuse de la chambre antérieure de l'oeil et/ou du corps vitré de l'oeil.

2. Lentille intraoculaire selon la revendication 1, **caractérisée en ce que** le corps de cristallin est constitué d'un matériau polymère, qui est réalisé d'au moins un acrylate hydrophobe et/ou d'un méthacrylate hydrophobe ou de caoutchouc de silicone.

3. Lentille intraoculaire selon la revendication 1 ou 2, **caractérisée en ce que** la couche hydrophile (2) est réalisée d'acrylate hydrophile.

4. Lentille intraoculaire selon l'une des revendications 1 à 3, **caractérisée en ce que** le corps de cristallin (1) présente au niveau de sa surface une micro-rugosité résultant de la fabrication de lentille par usinage ou du moulage (molding) de la lentille.

5. Lentille intraoculaire selon l'une des revendications 1 à 4, **caractérisée en ce que** des molécules de la couche hydrophile sont incorporées à une couche de surface du corps de cristallin (1).

6. Lentille intraoculaire selon la revendication 5, **caractérisée en ce que** les molécules incorporées de la couche hydrophile sont réticulées avec les molécules du polymère du corps de cristallin.

7. Lentille intraoculaire selon la revendication 5 ou 6, **caractérisée en ce que** la couche de surface du corps de cristallin, dans lequel les molécules de la couche hydrophile sont incorporées, présente un indice de réfraction situé entre les indices de réfraction de la couche hydrophile (2) et du corps de cristallin (1).

8. Lentille intraoculaire selon l'une des revendications 1 à 7, **caractérisée en ce que** la couche hydrophile (2) peut être expansée par l'humeur aqueuse et l'indice de réfraction de la zone superficielle de la couche hydrophile (2) est situé entre les indices de réfraction de l'humeur aqueuse et de la couche hydrophile (2).

9. Lentille intraoculaire selon l'une des revendications 1 à 8, **caractérisée en ce que** l'acrylate hydrophile est un méthacrylate dihydropropyl.

10. Appareil d'injection, qui présente un injecteur (3), avec lequel une lentille intraoculaire selon l'une des revendications 1 à 9 est à implanter, l'injecteur présentant au moins au niveau de sa paroi interne, le long de laquelle la lentille intraoculaire est guidée lors de l'implantation, un revêtement hydrophile (4), et la lentille intraoculaire étant un élément constitutif de l'appareil d'injection.

11. Appareil d'injection selon la revendication 10, **caractérisée en ce que** le revêtement hydrophile est un revêtement en acrylate.
